# FASCICULE DE BREVET EUROPEEN

(11) **EP 1 403 265 B1**
(45) Date de publication et mention de la délivrance du brevet: **25.11.2009**
(21) Numéro de dépôt: 03292318.7
(22) Date de dépôt: 22.09.2003
(51) Int. Cl.: C07D 333/38

(54) **Nouveau procédé de synthèse industriel des tétraesters de l'acide 5-¬bis(carboxyméthyl)amino -3-carboxyméthyl-4-cyano-2-thiophènecarboxylique, et application à la synthèse des sels bivalents de l'acide ranélique et de leurs hydrates**
Industrielles Verfahren zur Herstellung von 5-¬bis(carboxymethyl)amino -3-carboxymethyl-4-cyano-2-thiophencarbonsäure-tetraestern und Verwendung zur Synthese von zweiwertigen Ranetansäuresalzen und deren Hydraten
Industrial process for the synthesis of 5-¬bis(carboxymethyl)amino -3-carboxymethyl-4-cyano-2-thiophene-carboxylic acid tetraesters and use for the synthesis of ranetic acid divalent salts and hydrates thereof

(30) Priorité: 24.09.2002 FR 0211765
(43) Date de publication de la demande: 31.03.2004
(73) Titulaire: Les Laboratoires Servier, 92415 Courbevoie Cedex (FR)
(72) Inventeur: Vaysse-Ludot, Lucile, 76490 Saint Wandrille Rancon (FR); Lecouve, Jean-Pierre, 76600 Le Havre (FR); Langlois, Pascal, 76210 Saint Jean de la Neuville (FR)

(56) Documents cités:
- EP-A- 0 415 850
- EP-A- 0 813 869
- WIERZBICKI M ET AL: "Réactivité des amino-2 thiophènes. Application à la synthèse de quelques thiéno[2,3-b]pyrroles" BULLETIN DE LA SOCIETÉ CHIMIQUE DE FRANCE, MASSON, PARIS, FR, no. 7-8, 1975, pages 1786-1792, XP009008611 ISSN: 0037-8968

## Description

La présente invention concerne un procédé de synthèse industriel des tétraesters de l'acide 5-[bis(carboxyméthyl)amino]-3-carboxyméthyl-4-cyano-2-thiophènecarboxylique, et leur application à la production industrielle des sels bivalents de l'acide ranélique et de leurs hydrates.

Plus spécifiquement, la présente invention concerne un nouveau procédé de synthèse industriel des dérivés de formule (I) : 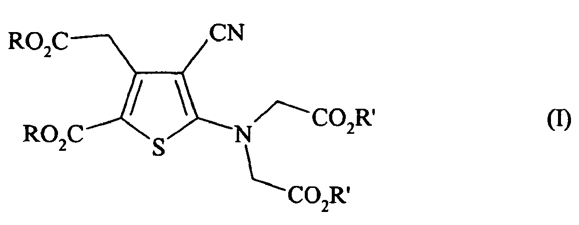 dans laquelle R et R', identiques ou différents, représentent chacun un groupement alkyle (C₁-C₆) linéaire ou ramifié.

Les composés de formule (I) obtenus selon le procédé de l'invention sont utiles dans la synthèse de l'acide ranélique, de ses sels de strontium, de calcium ou de magnésium de formule (II) : 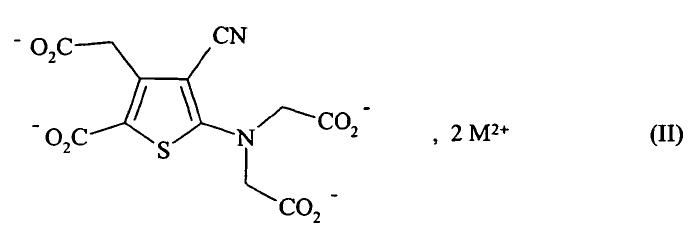 dans laquelle M représente le strontium, le calcium ou le magnésium,
et des hydrates desdits sels.

Les sels bivalents de l'acide ranélique possèdent des propriétés pharmacologiques et thérapeutiques très intéressantes, notamment des propriétés anti-ostéoporotiques remarquables, qui rendent ces composés utiles dans le traitement des maladies osseuses.

Les sels bivalents de l'acide ranélique, et plus particulièrement le ranélate de strontium, sa préparation et son utilisation en thérapeutique ont été décrits dans le brevet européen EP 0415 850.

Compte tenu de l'intérêt pharmaceutique de ce composé, il était important de pouvoir accéder à l'intermédiaire de formule (I) avec un procédé de synthèse industriel performant, permettant l'obtention du composé de formule (I) avec un bon rendement et une excellente pureté, mais également facilement transposable à l'échelle industrielle.

Le journal Bull. Soc. Chim. France 1975, pp. 1786-1792, décrit l'obtention d'un dérivé de formule (I) (R = R' = éthyle) par réaction de l'acide 5-amino-3-(carboxyméthyl)-4-cyano-2-thiophènecarboxylique avec le bromoacétate d'éthyle, en présence de carbonate de potassium, suivie d'un isolement en milieu hydro-organique très dilué.

Cependant, le faible rendement de cette réaction (65 %), la grande quantité de rejets aqueux salins générée par cette réaction, et surtout le temps de réaction très important (5 jours), étaient totalement dissuasifs quant à l'utilisation de cette réaction à l'échelle industrielle.

La Demanderesse a présentement mis au point un procédé de synthèse industriel simple, permettant d'obtenir le composé de formule (I) avec un très bon rendement, un temps de réaction considérablement plus court et une pureté excellente, et dans lequel les rejets aqueux salins sont complètement supprimés.

Plus spécifiquement, la présente invention concerne un procédé de synthèse industriel des composés de formule (I),
caractérisé en ce que l'on met en réaction un composé de formule (III) : 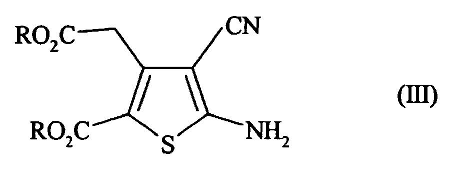 dans laquelle R représente un groupement alkyle (C₁-C₆) linéaire ou ramifié,
avec un composé de formule (IV) : 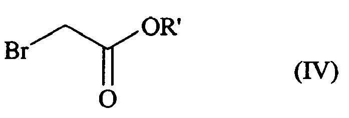 dans laquelle R' représente un groupement alkyle (C₁-C₆) linéaire ou ramifié,
en présence d'une quantité catalytique d'un ammonium quaternaire de type C₈-C₁₀,
et de carbonate de potassium,
au reflux d'un solvant organique,
que l'on filtre ensuite le mélange réactionnel,
puis que l'on concentre le milieu par distillation,
que l'on ajoute ensuite un cosolvant,
refroidit et filtre le mélange réactionnel,
pour conduire, après séchage de la poudre ainsi obtenue, au composé de formule (I).

Par ammonium quaternaire de type C₈-C₁₀, on entend un composé de formule (A) ou un mélange de composés de formule (A) :

R₁R₂R₃R₄-N^{+ -}X (A)

dans laquelle R₁ représente un groupement alkyle (C₁-C₆), R₂, R₃ et R₄, identiques ou différents, représentent chacun un groupement alkyle (C₈-C₁₀), et X représente un atome d'halogène.

Les ammoniums quaternaires de type C₈-C₁₀ préférés sont les catalyseurs Adogen 464® et Aliquat 336®.

De façon surprenante, seule l'utilisation d'un ammonium quaternaire de type C₈-C₁₀ permet l'obtention du composé de formule (I) à la fois avec un temps de réaction très réduit et avec une très bonne sélectivité, à la différence d'autres types d'ammoniums quaternaires, comme le montre le tableau suivant :

| **Catalyseur** | **Durée de réaction** | **Titre du milieu réactionnel** |
|---|---|---|
| Tétrabutylammoniumhydrogénosulfate (TBAHS) | 12 h | 92% |
| Bromure de N,N-bis(2-hydroxyéthyl)-N-méthyl 1-dodécanaminium | 18 h | 82% |
| Adogen 464^{®} | 5 h | 96 % |
| Aliquat 336^{®} | 4 h | 95 % |

De plus, l'isolement, pourtant simplifié (l'étape de précipitation suivie d'une filtration a été remplacée par une simple filtration du mélange réactionnel) permet, grâce aux conditions particulières qui ont été mises au point, d'obtenir le composé de formule (I), non seulement avec un très bon rendement (89 %), mais également avec une pureté excellente (supérieure à 98 %), et en supprimant la charge environementale que représentaient les rejets aqueux salins.
- La quantité de carbonate de potassium est préférentiellement comprise entre 2 et 3 moles par mole de composé de formule (III).
- La quantité de composé de formule (IV) est préférentiellement comprise entre 2 et 3 moles par mole de composé de formule (III).
- Le volume initial de solvant organique est préférentiellement compris entre 6 et 12 ml par gramme de composé de formule (III).
- Les solvants organiques préférés pour la réaction sont l'acétone et l'acétonitrile.
- Le cosolvant préféré pour l'isolement est le méthanol.

Le 5-[bis(2-méthoxy-2-oxoéthyl)amino]-4-cyano-3-(2-méthoxy-2-oxoéthyl)-2-thiophène carboxylate de méthyle et le 5-[bis(2-éthoxy-2-oxoéthyl)amino]-4-cyano-3-(2-méthoxy-2-oxoéthyl)-2-thiophènecarboxylate de méthyle, cas particuliers et préférés des composés de formule (I), sont des produits nouveaux, utiles comme intermédiaires de synthèse dans l'industrie chimique ou pharmaceutique, notamment dans la synthèse du ranélate de strontium, et font à ce titre partie intégrante de la présente invention.

Les exemples ci-dessous illustrent l'invention, mais ne la limitent en aucune façon.

### EXEMPLE 1 : 5-[Bis(2-méthoxy-2-oxoéthyl)amino]-4-cyano-3-(2-méthoxy-2-oxoéthyl)-2-thiophènecarboxylate de méthyle.

Dans un réacteur, charger 400 kg de l'ester diméthylique de l'acide 5-amino-3-(carboxyméthyl)-4-cyano-2-thiophènecarboxylique, 478 kg de carbonate de potassium, 2810 1 d'acétone, 16 kg d'Adogen 464® et 529,6 kg de bromoacétate de méthyle.

Amener la température à 60°C. Après 5 h de reflux, refroidir le mélange réactionnel, puis le filtrer. Concentrer le filtrat obtenu.

Ajouter du méthanol, refroidir et filtrer la suspension obtenue, puis sécher la poudre.

Le 5-[bis(2-méthoxy-2-oxoéthyl)amino]-4-cyano-3-(2-méthoxy-2-oxoéthyl)-2-thiophène-carboxylate de méthyle est ainsi obtenu avec un rendement supérieur à 85 % et une pureté chimique supérieure à 98 %.

### EXEMPLE 2 : 5-[Bis(2-méthoxy-2-oxoéthyl)amino]-4-cyano-3-(2-méthoxy-2-oxoéthyl)-2-thiophènecarboxylate de méthyle.

Le 5-[bis(2-méthoxy-2-oxoéthyl)amino]-4-cyano-3-(2-méthoxy-2-oxoéthyl)-2-thiophène carboxylate de méthyle est obtenu de la même façon que dans l'exemple 1, en remplaçant l'Adogen 464® par l'Aliquat 336® .

### EXEMPLE 3 : 5-[Bis(2-méthoxy-2-oxoéthyl)amino]-4-cyano-3-(2-méthoxy-2-oxoéthyl)-2-thiophènecarboxylate de méthyle.

Le 5-[bis(2-méthoxy-2-oxoéthyl)amino]-4-cyano-3-(2-méthoxy-2-oxoéthyl)-2-thiophène carboxylate de méthyle est obtenu de la même façon que dans l'exemple 1, en remplaçant l'acétone par l'acétonitrile.

### EXEMPLE 4: 5-[Bis(2-éthoxy-2-oxoéthyl)amino]-4-cyano-3-(2-méthoxy-2-oxoéthyl)-2-thiophènecarboxylate de méthyle.

Le 5-[bis(2-éthoxy-2-oxoéthyl)amino]-4-cyano-3-(2-méthoxy-2-oxoéthyl)-2-thiophène carboxylate de méthyle est obtenu de la même façon que dans l'exemple 1, en remplaçant les 529,6 kg de bromoacétate de méthyle par 578,1 kg de bromoacétate d'éthyle.

## Revendications

1. Procédé de synthèse industriel des composés de formule (I) : 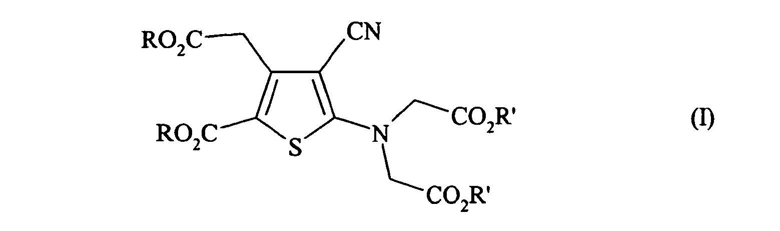 dans laquelle R et R', identiques ou différents, représentent chacun un groupement alkyle (C₁-C₆) linéaire ou ramifié,
**caractérisé en ce que** l'on met en réaction un composé de formule (III) : 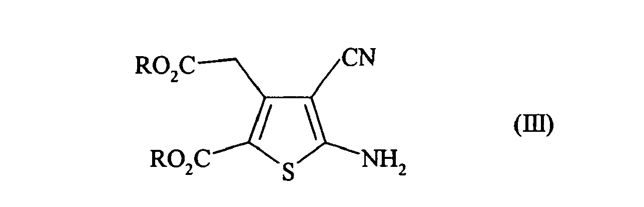 dans laquelle R est tel que défini précédemment,
avec un composé de formule (IV) 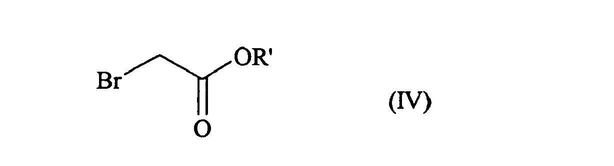 dans laquelle R' est tel que défini précédemment,
en présence d'une quantité catalytique d'un ammonium quaternaire de type C₈-C₁₀,
et de carbonate de potassium,
au reflux d'un solvant organique,
que l'on filtre ensuite le mélange réactionnel,
puis que l'on concentre le milieu par distillation,
que l'on ajoute ensuite un cosolvant,
refroidit et filtre le mélange réactionnel,
pour conduire, après séchage de la poudre ainsi obtenue, au composé de formule (I),
étant entendu que par ammonium quaternaire de type C₈-C₁₀, on entend un composé de formule (A) ou un mélange de composés de formule (A) :
R₁ R₂ R₃ R₄-N⁺⁻X (A)
dans laquelle R₁ représente un groupement alkyle (C₁-C₆), R₂, R₃ et R₄, identiques ou différents, représentent chacun un groupement alkyle (C₈-C₁₀), et X représente un atome d'halogène.

2. Procédé de synthèse selon la revendication 1, permettant l'obtention du dérivé de formule (I) dans laquelle R représente le groupement méthyle, et R' représente le groupement éthyle.

3. Procédé de synthèse selon la revendication 1, permettant l'obtention du dérivé de formule (I) dans laquelle R et R' représentent chacun le groupement méthyle.

4. Procédé selon l'une quelconque des revendications 1 à 3, **caractérisé en ce que** l'ammonium quaternaire de type C₈-C₁₀ est l'Adogen 464^{®} ou l'Aliquat 336^{®}.

5. Procédé de synthèse selon l'une quelconque des revendications 1 à 4, **caractérisé en ce que** la quantité de carbonate de potassium est comprise entre 2 et 3 moles par mole de composé de formule (III).

6. Procédé de synthèse selon l'une quelconque des revendications 1 à 5, **caractérisé en ce que** la quantité de composé de formule (IV) est comprise entre 2 et 3 moles par mole de composé de formule (III).

7. Procédé de synthèse selon l'une quelconque des revendications 1 à 6, **caractérisé en ce que** le volume initial de solvant organique est compris entre 6 et 12 ml par gramme de composé de formule (III).

8. Procédé de synthèse selon l'une quelconque des revendications 1 à 7, **caractérisé en ce que** le solvant organique utilisé pour la réaction est l'acétone ou l'acétonitrile.

9. Procédé de synthèse selon l'une quelconque des revendications 1 à 8, **caractérisé en ce que** le cosolvant utilisé lors de l'isolement est le méthanol.

10. Procédé de synthèse selon l'une quelconque des revendications 1 à 9, **caractérisé en ce que** le composé de formule (I) obtenu a une pureté chimique supérieure à 98 %.

11. 5-[Bis(2-méthoxy-2-oxoéthyl)amino]-4-cyano-3-(2-méthoxy-2-oxoéthyl)-2-thiophènecarboxylate de méthyle.

12. 5-[Bis(2-éthoxy-2-oxoéthyl)amino]-4-cyano-3-(2-méthoxy-2-oxoéthyl)-2-thiophènecarboxylate de méthyle.

13. Procédé de synthèse de l'acide ranélique, de ses sels de strontium, de calcium ou de magnésium et des hydrates desdits sels, à partir d'un composé de formule (I) : 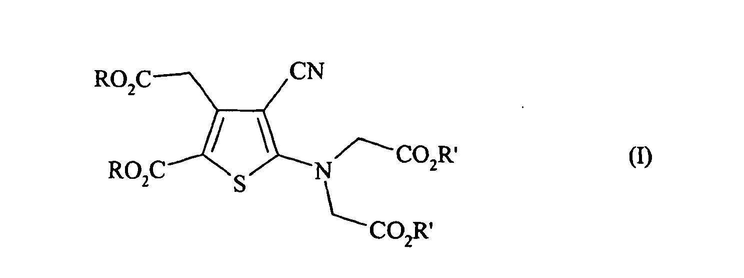 dans laquelle R et R', identiques ou différents, représentent chacun un groupement alkyle (C₁-C₆) linéaire ou ramifié,
**caractérisé en ce que** le composé de formule (I) est obtenu par le procédé de synthèse selon l'une quelconque des revendications 1 à 10.

14. Procédé de synthèse du ranélate de strontium et de ses hydrates à partir d'un composé de formule (I) : 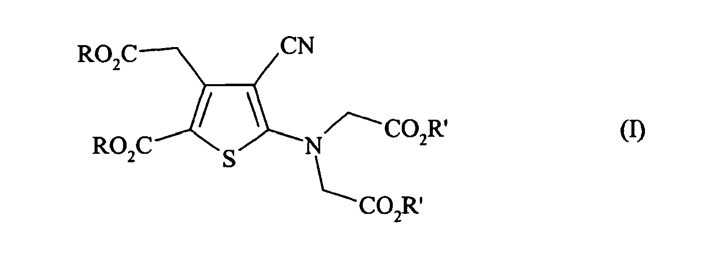 dans laquelle R et R', identiques ou différents, représentent chacun un groupement alkyle (C₁-C₆) linéaire ou ramifié,
**caractérisé en ce que** le composé de formule (I) est obtenu par le procédé de synthèse selon l'une quelconque des revendications 1 à 10.

## Claims

1. Process for the industrial synthesis of compounds of formula (I) : 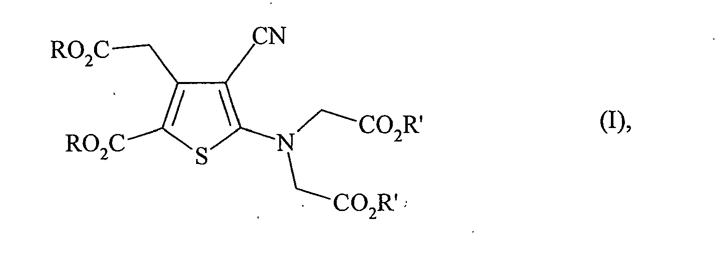 wherein R and R', which are the same or different, each represent a linear or branched (C₁-C₆)alkyl group,
**characterised in that** a compound of formula (III) : 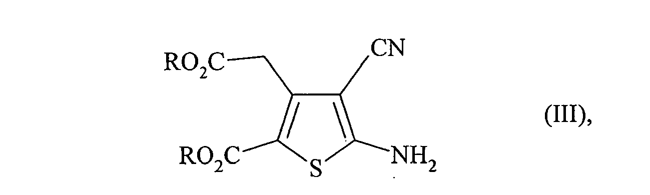 wherein R is as defined hereinbefore,
is reacted with a compound of formula (IV) : 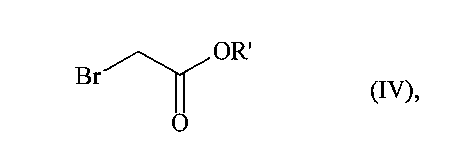 wherein R' is as defined hereinbefore,
in the presence of a catalytic amount of a C₈-C₁₀-type quaternary ammonium compound,
and in the presence of potassium carbonate,
at the reflux of an organic solvent;
the reaction mixture is subsequently filtered;
the mixture is then concentrated by distillation;
a co-solvent is then added,
and the reaction mixture is cooled and filtered
to yield, after drying of the powder thereby obtained, the compound of formula (I),
it being understood that a C₈-C₁₀-type quaternary ammonium compound is a compound of formula (A) or a mixture of compounds of formula (A) :
R₁ R₂ R₃ R₄-N ⁺⁻ X (A)
wherein R₁ represents a (C₁-C₆)alkyl group, R₂, R₃ and R₄, which are the same or different, each represent a (C₈-C₁₀)alkyl group, and X represents a halogen atom.

2. Synthesis process according to claim 1 allowing the compound of formula (I), wherein R represents a methyl group and R' represents an ethyl group, to be obtained.

3. Synthesis process according to claim 1 allowing the compound of formula (I), wherein R and R' each represent a methyl group, to be obtained.

4. Process according to any one of claims 1 to 3, **characterised in that** the C₈-C₁₀-type quaternary ammonium compound is Adogen 464^{®} or Aliquat 336^{®}.

5. Synthesis process according to any one of claims 1 to 4, **characterised in that** the amount of potassium carbonate is from 2 to 3 mol per mol of compound of formula (III).

6. Synthesis process according to any one of claims 1 to 5, **characterised in that** the amount of compound of formula (IV) is from 2 to 3 mol per mol of compound of formula (III).

7. Synthesis process according to any one of claims 1 to 6, **characterised in that** the initial volume of organic solvent is from 6 to 12 ml per gram of compound of formula (III).

8. Synthesis process according to any one of claims 1 to 7, **characterised in that** the organic solvent used for the reaction is acetone or acetonitrile.

9. Synthesis process according to any one of claims 1 to 8, **characterised in that** the co-solvent used during isolation is methanol.

10. Synthesis process according to any one of claims 1 to 9, **characterised in that** the compound of formula (1) obtained has a chemical purity greater than 98 %.

11. Methyl 5-[bis(2-methoxy-2-oxoethyl)amino]-4-cyano-3-(2-methoxy-2-oxoethyl)-2-thiophenecarboxylate.

12. Methyl 5-[bis(2-ethoxy-2-oxoethyl)amino]-4-cyano-3-(2-methoxy-2-oxoethyl)-2-thiophenecarboxylate.

13. Process for the synthesis of ranelic acid, its strontium, calcium or magnesium salts and hydrates of the said salts, starting from a compound of formula (I) : 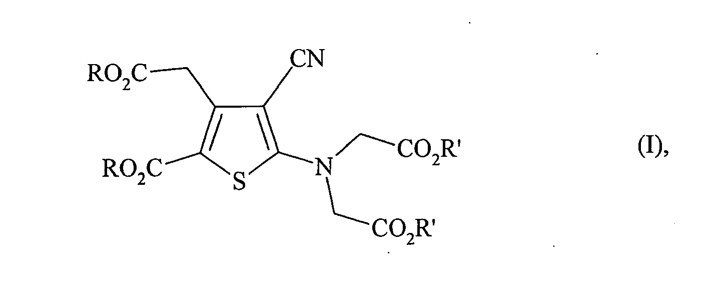 wherein R and R', which are the same or different, each represent a linear or branched (C₁-C₆)alkyl group,
**characterised in that** the compound of formula (I) is obtained by the synthesis process according to any one of claims 1 to 10.

14. Process for the synthesis of strontium ranelate and its hydrates, starting from a compound of formula (I) : 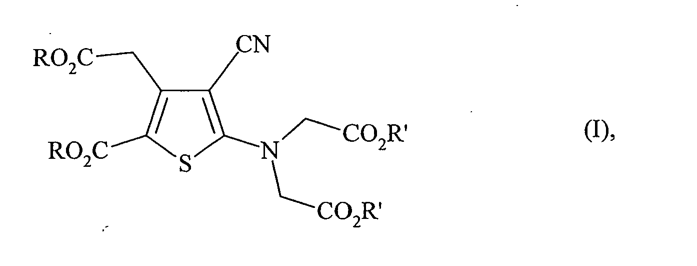 wherein R and R', which are the same or different, each represent a linear or branched (C₁-C₆)alkyl group,
**characterised in that** the compound of formula (I) is obtained by the synthesis process according to any one of claims 1 to 10.

## Patentansprüche

1. Verfahren zur technischen Synthese der Verbindungen der Formel (I): 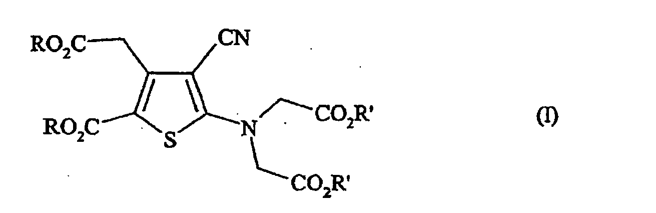 in der R und R', die identisch oder verschieden sind, jeweils eine geradkettige oder verzweigte (C₁-C₆)-Alkylgruppe bedeuten,
**dadurch gekennzeichnet, dass** man eine Verbindung der Formel (III): 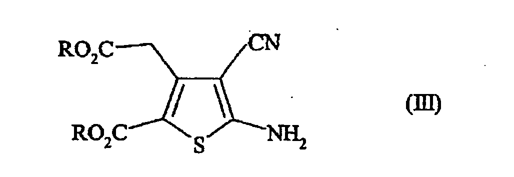 in der R die oben angegebenen Bedeutungen besitzt,
mit einer Verbindung der Formel (IV) 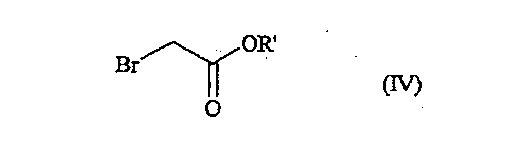 in der R' die oben angegebenen Bedeutungen besitzt,
in Gegenwart einer katalytischen Menge einer quaternären Ammoniumverbindung des Typs C₈-C₁₀ und von Kaliumcarbonat
am Rückfluss in einem organischen Lösungsmittel umsetzt,
anschließend die Reaktionsmischung filtriert,
dann das Medium durch Destillation einengt,
anschließend ein Co-Lösungsmittel zusetzt und
die Reaktionsmischung abkühlt und filtriert,
so das man nach dem Trocknen des in dieser Weise erhaltenen Pulvers die Verbindung der Formel (I) erhält
mit der Maßgabe, dass man unter einer quaternären Ammoniumverbindung des Typs C₈-C₁₀ eine Verbindung der Formel (A) oder eine Mischung von Verbindungen der Formel (A) versteht:
R₁R₂R₃R₄ -N⁺⁻X (A)
in der R₁ eine (C₁-C₆)-Alkylgruppe bedeutet, R₂, R₃ und R₄, die identisch oder verschieden sind, jeweils eine (C₈-C₁₀)-Alkylgruppe bedeuten und X ein Halogenatom bedeutet.

2. Syntheseverfahren nach Anspruch 1 für die Herstellung des Derivats der Formel (I), in der R die Methylgruppe bedeutet und R' die Ethylgruppe bedeutet.

3. Syntheseverfahren nach Anspruch 1 für die Herstellung des Derivats der Formel (I), in der R und R' jeweils die Methylgruppe bedeuten.

4. Verfahren nach einem der Ansprüche 1 bis 3, **dadurch gekennzeichnet, dass** die quaternäre Ammoniumverbindung des Typs C₈-C₁₀ Adogen 464^{®} oder Aliquat 336^{®} ist.

5. Syntheseverfahren nach einem der Ansprüche 1 bis 4, **dadurch gekennzeichnet, dass** die Menge von Kaliumcarbonat zwischen 2 und 3 Mol pro Mol der Verbindung der Formel (III) beträgt.

6. Syntheseverfahren nach einem der Ansprüche 1 bis 5, **dadurch gekennzeichnet, dass** die Menge der Verbindung der Formel (IV) zwischen 2 und 3 Mol pro Mol der Verbindung der Formel (III) beträgt.

7. Syntheseverfahren nach einem der Ansprüche 1 bis 6, **dadurch gekennzeichnet, dass** das Anfangsvolumen des organischen Lösungsmittels zwischen 6 und 12 ml pro Gramm der Verbindung der Formel (III) beträgt.

8. Syntheseverfahren nach einem der Ansprüche 1 bis 7, **dadurch gekennzeichnet, dass** das für die Reaktion verwendete organische Lösungsmittel Aceton oder Acetonitril ist.

9. Syntheseverfahren nach einem der Ansprüche 1 bis 8, **dadurch gekennzeichnet, dass** das bei der Isolierung verwendete Co-Lösungsmittel Methanol ist.

10. Syntheseverfahren nach einem der Ansprüche 1 bis 9, **dadurch gekennzeichnet, dass** die erhaltene Verbindung der Formel (I) eine chemische Einheit von mehr als 98 % aufweist.

11. 5-[Bis(2-methoxy-2-oxoethyl)-amino]-4-cyano-3-(2-methoxy-2-oxoethyl)-2-thiophencarbonsäuremethylester.

12. 5-[Bis(2-ethoxy-2-oxoethyl)-amino]-4-cyano-3-(2-methoxy-2-oxoethyl)-2-thiophencarbonsäuremethylester.

13. Verfahren zur Synthese von Ranelinsäure, seinen Strontiumsalzen, Calciumsalzen oder Magnesiumsalzen und den Hydraten dieser Salze ausgehend von einer Verbindung der Formel (I): 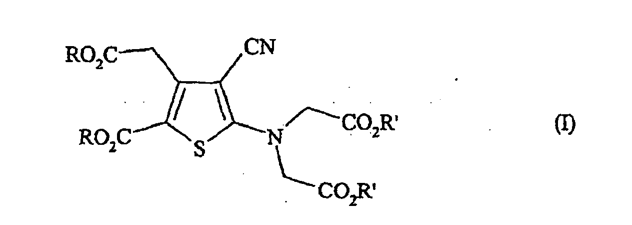 in der R und R', die identisch oder verschieden sind, jeweils eine geradkettige oder verzweigte (C₁-C₆)-Alkylgruppe bedeuten,
**dadurch gekennzeichnet, dass** die Verbindung der Formel (I) nach dem Syntheseverfahren nach einem der Ansprüche 1 bis 10 erhalten wird.

14. Verfahren zur Synthese von Strontiumranelat und von seinen Hydraten ausgehend von einer Verbindung der Formel (I): 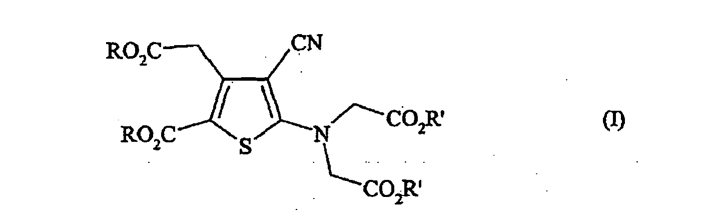 in der R und R', die identisch oder verschieden sind, jeweils eine geradkettige oder verzweigte (C₁-C₆)-Alkylgruppe bedeuten,
**dadurch gekennzeichnet, dass** die Verbindung der Formel (I) nach dem Syntheseverfahren nach einem der Ansprüche 1 bis 10 erhalten wird.
